# EUROPÄISCHE PATENTANMELDUNG

(11) **EP 4 156 707 A1**
(43) Veröffentlichungstag der Anmeldung: **29.03.2023**
(21) Anmeldenummer: 22195675.8
(22) Anmeldetag: 14.09.2022
(51) Int. Cl.: H04R 1/08, G01L 1/00, G01D 11/24, G01N 33/00, G01H 11/00, G01L 19/14

(54) **ZUGANGSELEMENT FÜR MEDIEN AN EINEM SENSOR SOWIE SENSOR MIT EINEM DERARTIGEN ZUGANGSELEMENT**

(30) Priorität: 23.09.2021 DE 102021210581
(71) Anmelder: Robert Bosch GmbH, 70442 Stuttgart (DE)
(72) Erfinder: Seitz, Roland, 72147 Nehren (DE); Woernle, Wolfgang, 72149 Neustetten (DE); Merz, Andreas, 71691 Freiberg Am Neckar (DE); Muehlberger-Koza, Yoshie, 70176 Stuttgart (DE); Hortig, Michael, 72800 Eningen U. A. (DE); Buck, Thomas, 71732 Tamm (DE)

(57) **Zusammenfassung**

Mit der vorliegenden Erfindung wird ein Zugangselement eines Sensors sowie ein Sensor mit einem derartigen Zugangselement beansprucht. Das Zugangselement ist dabei dazu eingerichtet, das Medium, zum Beispiel ein Gas oder eine Flüssigkeit an ein im Sensor befindliches Sensorelement zu leiten. Das erfindungsgemäße Zugangselement ist derart ausgestaltet, dass es in oder an einem Medienkanal des Sensors angeordnet sein kann. Hierzu kann sowohl das Zugangselement als auch der Sensor entsprechende Befestigungselemente aufweisen. Weiterhin ist vorgesehen, dass das Zugangselement einen Hohlzylinder aufweist, der eine obere erste Öffnung aufweist, durch die das Medium eindringen und/oder durch den Hohlzylinder an das Sensorelement durchgeleitet werden kann. Der Hohlzylinder weist darüber hinaus ein Abdeckelement auf, welches über zwei Stege insbesondere mit der Innenwand oder dem äußeren oberen oder unteren Rand des Hohlzylinders verbunden ist. Zur Verhinderung des Eindringens von störenden Teilchen ist eine konische Ausformung im Inneren des Hohlzylinders vorgesehen, welche von der Innenwand des Hohlzylinders ins Innere weist und eine zweite Öffnung aufweist, die kleiner als die erste Öffnung ist.

## Beschreibung

Die Erfindung betrifft ein Medien-Zugangselement an oder in einem Sensor sowie einen Sensor, der ein solches Zugangselement aufweist.

### Stand der Technik

Um physikalische und/oder chemische Größen eines Mediums zu erfassen, ist bei den meisten Sensoren ein direkten Medienzugang zum Sensorelement notwendig. Dabei besteht die Gefahr, dass durch Eindringende Teilchen, Staub oder Dreck in den Medienzugang direkt oder indirekt das Sensorelement beschädigt wird oder zumindest die Messwertefassung beeinträchtigt werden kann. Gerade bei Sensorarten, die keine vollständige Abkapselung gegenüber der Umgebung erlauben, wie beispielsweise einem Drucksensor, einem Gassensor oder einem Mikrofon kann es daher zu einem Absetzung von Staub oder Dreck sowohl im Medienzugang als auch auf dem Sensorelement kommen. Darüber hinaus besteht ebenfalls die Gefahr, dass unerwünschte Teilchen wie Steine in den Medienzugang eintreten und im extremsten Fall das Sensorelement beschädigen. Verbleiben diese Teilchen im Medienkanal können sie beispielsweise das Schwingungsverhalten des Sensors und somit die Erfassung der Sensorgröße nachteilig verändern.

Zur Verhinderung des Eindringens von unerwünschten Elementen oder Teilchen bei einem Mikrofon ist aus der DE 100 46 874 A1 ein Mikrofongehäuse mit einem Feuchtigkeits- und Staubschutz bekannt. Zu diesem Zweck wird ein Gehäuse bereitgestellt, welches eine Aufnahme zum Einschieben des eigentlichen Mikrofons aufweist. Die Aufnahme besitzt dabei einen schalldurchlässigen Bereich, der als Gitter ausgebildet ist.

Aufgabe der vorliegenden Erfindung ist es, einen erweiterten Schutz für einen Sensor bereitzustellen, der das Eindringen von kleinen und größeren störenden Teilchen verhindert, ohne die Erfassung der Sensorgröße zu behindern.

### Offenbarung der Erfindung

Mit der vorliegenden Erfindung wird ein Zugangselement eines Sensors sowie ein Sensor mit einem derartigen Zugangselement beansprucht. Das Zugangselement ist dabei dazu eingerichtet, das Medium, zum Beispiel ein Gas oder eine Flüssigkeit an ein im Sensor befindliches Sensorelement zu leiten. Dabei kann auch vorgesehen sein, dass lediglich eine physikalische und/oder chemische Größe des Mediums an das Sensorelement geleitet wird, zum Beispiel ein Druck oder ein Schall. Das Sensorelement kann mittels mikromechanischer Verfahren hergestellt sein.

Das erfindungsgemäße Zugangselement ist derart ausgestaltet, dass es in oder an einem Medienkanal des Sensors angeordnet sein kann. Hierzu kann sowohl das Zugangselement als auch der Sensor entsprechende Befestigungselemente aufweisen. Weiterhin ist vorgesehen, dass das Zugangselement einen Hohlzylinder aufweist, der eine obere erste Öffnung aufweist, durch die das Medium eindringen und/oder durch den Hohlzylinder an das Sensorelement durchgeleitet werden kann. Der Hohlzylinder weist darüber hinaus ein Abdeckelement auf, welches über zwei Stege insbesondere mit der Innenwand oder dem äußeren oberen oder unteren Rand des Hohlzylinders verbunden ist. Zur Verhinderung des Eindringens von störenden Teilchen ist eine konische Ausformung im Inneren des Hohlzylinders vorgesehen, welche von der Innenwand des Hohlzylinders ins Innere weist und eine zweite Öffnung aufweist, die kleiner als die erste Öffnung ist.

Mit der vorstehende beschriebenen Ausgestaltung des Zugangselements als Hohlzylinder kann erreicht werden, dass größere Teilchen wie Steine nicht eindringen können oder zumindest nicht gänzlich durch den Hohlzylinder hindurch bis auf das Sensorelement hindurchtreten können. Weiterhin wird durch die konische Ausgestaltung eine Ablagerungsfläche geschaffen, auf der sich Staub oder Dreck ablagern kann, ohne dass sich der Staub oder der Dreck direkt auf das Sensorelement legt.

In einer besonderen Ausgestaltung ist vorgesehen, dass der Austritt des Hohlzylinders im unterer Teil des Hohlzylinders eine dritte Öffnung aufweist, die in Richtung des Sensorelements gerichtet ist. Dabei kann die zweite Öffnung, die durch die konische Ausgestaltung vorgegeben ist, den gleichen Durchmesser wie der Austritt des Hohlzylinders aufweisen. Optional kann dabei vorgesehen sein, dass diese zweite Öffnung bündig mit dem Rand im unteren Bereich des unteren Teils des Hohlzylinders verbunden ist, zum Beispiel indem die konische Ausformung bis zum unteren Teil verlängert ausgebildet ist. Optional kann auch vorgesehen sein, dass die konische Ausformung nur im unteren Teil des Hohlzylinders ausgebildet ist.

Um die Größe der zurückgehaltenen Teile des Mediums zu definieren, kann das Verhältnis der Durchmesser des Abdeckelements zur ersten und/oder zweiten Öffnung gewählt werden. So kann ein Durchmesser des Abdeckelements, welches größer als der Durchmesser der zweiten Öffnung ist, verhindern, dass insbesondere kleinere Teilchen direkt durch den Hohlzylinder hindurchtreten und zunächst auf die konische Ausformung treffen. Durch die Wahl des Abstands des Abdeckelements zu zweiten Öffnung kann zudem festgelegt werden, welche Größe der Teilchen noch durch den Hohlzylinder hindurchtreten können. Optional kann das Abdeckelement unterhalb oder oberhalb der durch die konische Ausformung definierten zweiten Öffnung angeordnet sein.

In einer weiteren Ausführung kann auch vorgesehen sein, dass das Abdeckelement einen Durchmesser aufweist, der größer als die erste Öffnung im Hohlzylinder ist. In diesem Fall ist das Abdeckelement oberhalb des Hohlzylinders mittels Stegen zum Beispiel am Rand der Wandung des Hohlzylinders angebracht. Somit dient das Abdeckelement als Deckel, welcher über den Rand des Hohlzylinders hinaus ragt. Das Medium oder auch Schall kann dabei seitlich unter dem Abdeckelement in den Hohlzylinder des Zugangselements eintreten.

In einer besonderen Ausgestaltung der Erfindung ist vorgesehen, dass das Abdeckelement innerhalb der oberen ersten Öffnung des Hohlzylinders angeordnet ist. Dabei kann die Oberseite des Abdeckelements sowie insbesondere die Befestigungsstege bündig mit dem Rand des Hohlzylinders ausgestaltet sein.

Besonders vorteilhaft ist eine Anordnung des Abdeckelements zentral in der ersten, zweiten oder dritten Öffnung.

Der Hohlzylinder kann als Kreiszylinder, als elliptischer Zylinder oder in Form eines Prismas ausgestaltet sein. Dabei kann die Wandstärke insbesondere durch die konische Ausformung vom oberen Bereich zum unteren Bereich variieren.

In einer Weiterbildung der Erfindung ist vorgesehen, dass in oder an dem Hohlzylinder eine Membran angeordnet ist, die das Eindringen von Staub oder Dreck in den angeschlossenen Medienkanal des Sensors weiter verhindert. So kann die Membran am unteren Ende des Hohlzylinders angeordnet sein, indem diese die dritte Öffnung abdeckt. Optional kann die so angebrachte Membran direkt auf eine entsprechende Aufnahme eines Gehäusedeckels des Sensors aufgebracht werden.

Ein Sensor, der ein erfindungsgemäßes Zugangselement aufweist besteht beispielsweise aus einem Gehäuse, einem in dem Gehäuse angeordneten Sensorelement, einem Gehäusedeckel und dem Zugangselement. Der Gehäusedeckel, der optional auch Teil des Gehäuses sein kann, weist einen Medienkanal zur Weiterleitung des Mediums und/oder einer physikalischen beziehungsweise chemischen Größe des Mediums an das Sensorelement auf. Das Zugangselement ist derart am oder im Gehäusedeckel angeordnet beziehungsweise befestigt, dass der Hohlzylinder die Weiterleitung des Mediums an den Medienkanal erlaubt. Die obere Öffnung des Hohlzylinders ist vom Sensor weg gerichtet, während die untere dritte Öffnung vorzugsweise auf den Medienkanal und/oder das Sensorelement hin ausgerichtet ist.

In einer Ausführung kann vorgesehen sein, dass zwischen dem Zugangselement und dem Medienkanal eine Membran vorgesehen ist. Dabei kann die Membran im Wesentlichen am Zugangselement oder am Gehäusedeckel angebracht sein.

Für Abdichtungszwecke kann zwischen dem Zugangselement und dem Gehäusedeckel oder dem Medienkanal eine Dichtung vorgesehen sein, insbesondere eine Silikon-/Elastomerdichtung, die beispielsweise im Spritzgussverfahren vor dem Zusammenfügen der beiden Elemente aufgebracht wird. Diese Dichtung kann auch die Membran und den Gehäusedeckel miteinander verbinden und abdichten. Optional kann die Dichtung auch mechanische Schwingungen dämpfen, so dass ungewollte Störungen von extern nicht auf das Gehäuse und/oder das Sensorelement übertragen werden können.

Weitere Vorteile ergeben sich aus der nachfolgenden Beschreibung von Ausführungsbeispielen bzw. aus den abhängigen Patentansprüchen.

### Kurze Beschreibung der Zeichnungen

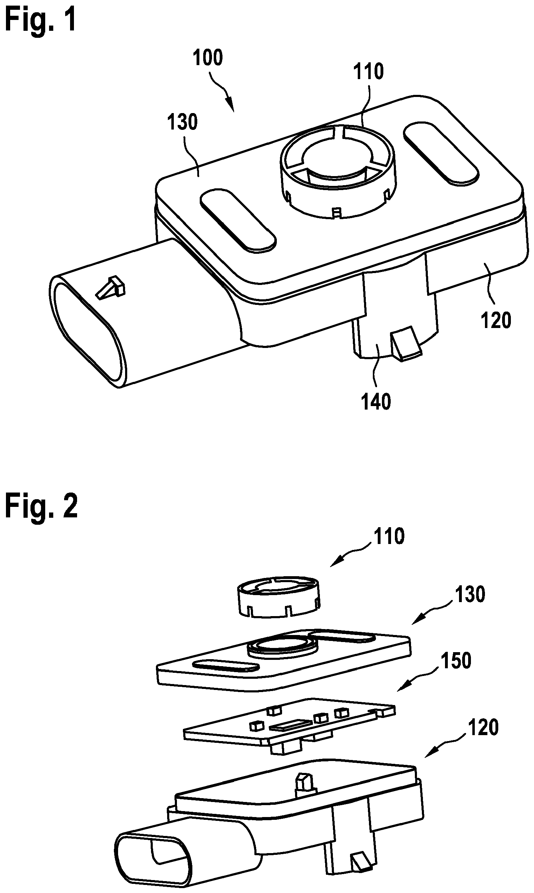

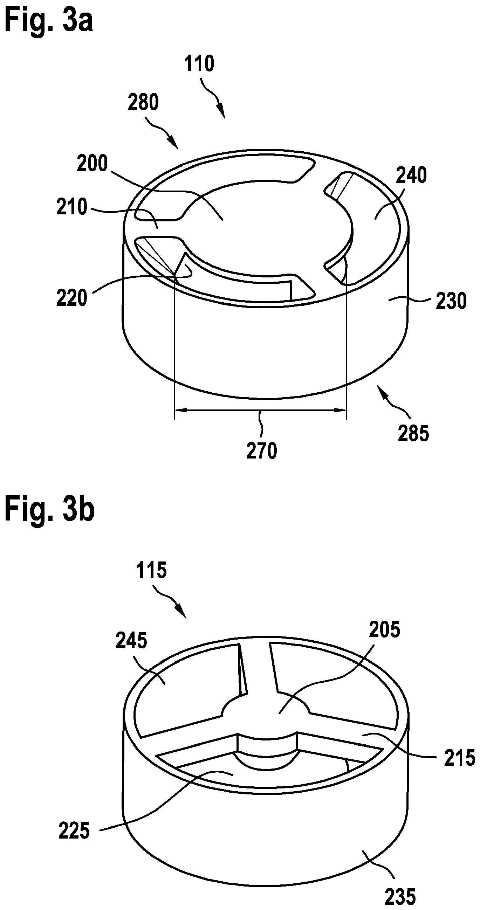

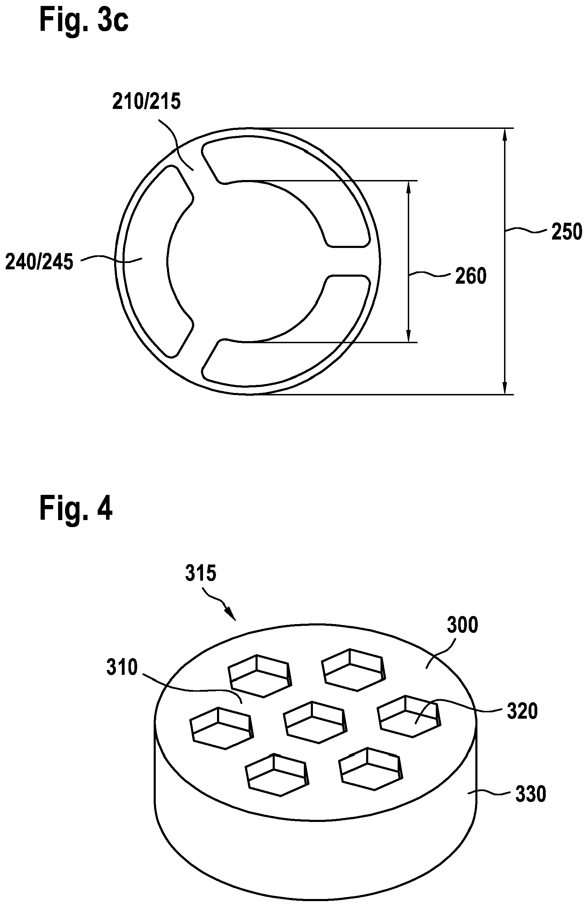

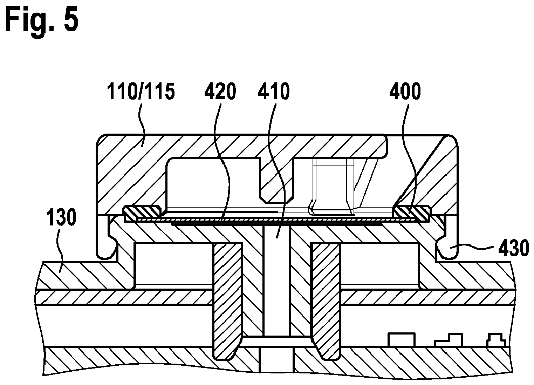

In der Figur 1 wird schematisch ein Aufbau eines Sensors mit einem erfindungsgemäßen Zugangselement gezeigt. Die Explosionszeichnung in Figur 2 zeigt die einzelnen Elemente des Aufbaus des Sensors. Die Figuren 3a bis c zeigen verschiedene Ansichten möglicher Zugangselemente. Mit der Figur 4 wird eine weitere mögliche Ausgestaltung des Zugangselements dargestellt. Eine Weiterentwicklung des Aufbaus aus der Figur 1 wird im Rahmen der Querschnittsdarstellung der Figur 5 beschrieben.

### Ausführungsformen der Erfindung

Sensorelemente, die physikalische und/oder chemische Größen eines Mediums erfassen, werden typischerweise in Sensoren 100 verwendet, wie sie in Figur 1 sowie in der Explosionszeichnung der Figur 2 dargestellt sind. Dabei wird das Sensorelement auf einer Leiterplatte 150 oder einem Träger in einem Gehäuse 120 untergebracht. Die Leiterplatte 150 kann zusätzlich auch Auswerteeinheiten aufweisen, mittels der das Sensorelement sowohl angesteuert als auch dessen Auswertung durchgeführt werden kann. Vorteilhafterweise werden dabei mikromechanisch erzeugte Sensorelement verwendet, um die räumlichen Dimensionen möglichst gering zu halten. Zur Abdeckung des Sensorelements kann der Sensor 100 einen separaten oder mit dem Gehäuse 120 verbundenen Gehäusedeckel 130 aufweisen. Dieser Gehäusedeckel weist eine Öffnung auf, durch die das Medium von der Umgebung des Sensors an das Sensorelement gelangen kann. Das erfindungsgemäße Zugangselement 110 ist dabei in oder an dieser Öffnung angeordnet. Wie aus dem Querschnitt einer noch zu beschreibenden Ausführungsform der Erfindung gemäß der Figur 5 zu erkennen ist, kann im Gehäusedeckel ein Medienkanal 410 ausgestaltet sein, auf welchen der Hohlzylinder, aus dem das Zugangselement 110 besteht, ausgerichtet ist. Das Zugangselement 110 kann mittels Klammern 430 zur Befestigung einen Vorsprung des Gehäusedeckels 130 umklammern. Darüber hinaus sind jedoch auch andere Befestigungen des Zugangselements 110 an oder in dem Gehäusedeckel 130 möglich.

Mögliche Ausgestaltungen des Zugangselements sind in den Figuren 3a bis c dargestellt. In einer ersten Varianten besteht das Zugangselements 110 aus einem kreisförmigen Hohlzylinder. Darüber hinaus ist jedoch auch jede andere Form eines Hohlzylinders möglich, der eine Durchleitung des Mediums erlaubt. Der Hohlzylinder besteht aus einer Wandung 230 mit einer ersten oberen Öffnung 250 (siehe Figur 3c), an deren Innenseite eine konische Ausformung 240 vorgesehen ist sowie einer unteren dritten Öffnung. Diese konische Ausformung 240 führt von der Innenwand der Wandung 230 nach innen und weist eine zweite Öffnung 270 auf, die einen kleineren Durchmesser als die erste Öffnung 250 aufweist. Um eine Eindringen von größeren Teilchen des Mediums oder auch von Steinchen zu vermeiden, ist ein Abdeckelement 200 vorgesehen, welches zentral in der ersten Öffnung 250 angeordnet ist. Zur Halterung dieses Abdeckelements 200, welches einen kleineren Durchmesser 260 als die erste Öffnung 250 aufweist, sind wenigstens zwei Stege 210 vorgesehen, die das Abdeckelement 200 an der Wandung 230 halten. In der Ausgestaltung des Zugangselements 110 gemäß der Figur 3a ist der Durchmesser 260 des Abdeckelements 200 größer als die durch die konische Ausformung 240 bereitgestellte zweite Öffnung 270. Durch eine derartige Ausgestaltung kann verhindert werden, dass Teile der Umgebung direkt durch den Hohlzylinder von oben 280 auf das Sensorelement oder in das Innere des Sensors 100 gelangen. Durch die Abstimmung sowohl der Durchmesser des Abdeckelements 200 und der zweiten Öffnung 270 als auch des Abstandes vom Abdeckelement 200 zum Ende der konischen Ausformung 240 mit der zweiten Öffnung 270 kann zusätzlich festgelegt werden, welche Teilchengröße vom Eindringen in den Hohlzylinder und insbesondere in den Medienkanal abgehalten werden. Eine weitere Möglichkeit, die eindringende Teilchengröße oder genauer gesagt, die Teilchengröße zu bestimmen, die durch die Gestaltung des Zugangselements abgehalten wird, besteht darin, die Dimensionen der Stege 210 und/oder der dazwischen liegenden Öffnungen 220 zu variieren.

Optional kann vorgesehen sein, dass die konische Ausformung 240 im oberen Bereich 280 des Hohlzylinders am Rand der Wandung 230 angebracht ist und von dieser nach Innen führt. Dabei kann ebenfalls vorgesehen sein, dass die konische Ausformung 240 bis zum Boden 285 des Hohlzylinders führt. Indem dort eine bündige Verbindung zwischen dem Rand der zweiten Öffnung 270 und dem unteren Rand der Wandung 240 vorgesehen ist, kann eine entsprechende Fläche für die Befestigung an dem Gehäusedeckel 120 bereitgestellt werden, zum Beispiel in Form einer Klebeverbindung. In diesem Fall entspricht der Durchmesser der dritte Öffnung der zweiten Öffnung 270.

Anhand der Figur 3b wird eine weitere Ausführung eines Zugangselements 115 mit einer Wandung 235 gezeigt, bei der der Durchmesser des Abdeckelements 205 kleiner als die zweite Öffnung der konischen Ausformung 245 ist. Die Stege 215 zur Halterung des Abdeckelements 205 können hierbei dicker ausgestaltet sein, um die Öffnungen 225 zu verkleinern.

In der Ausgestaltung der Figuren 3a und b ist die konische Ausformung im oberen Bereich des entsprechenden Hohlzylinders und insbesondere ausgehend von dessen oberen Rand ausgestaltet. Optional kann diese konische Ausformung jedoch auch tiefer innerhalb des Hohlzylinders angeordnet oder ausgestaltet sein.

Optional kann auch vorgesehen sein, dass das Abdeckelement 200 beziehungsweise 205 unterhalb der konischen Ausformung 240 oder 245 beziehungsweise der zweiten Öffnung 270 angeordnet sein.

In einer weiteren Ausgestaltung kann vorgesehen sein, dass das Abdeckelement oberhalb des oberen Randes des Zugangselement mit einem Abstand angeordnet ist. In diesem Fall kann das Abdeckelement einen Durchmesser aufweisen, welcher gleich oder größer als die erste Öffnung oder der der Durchmesser des Hohlzylinders ist. So kann das Abdeckelement über den Rand des Hohlzylinders hinaus ragen, so dass auch durch eine derartige Anordnung ein direktes Eintreten von Staub, Dreck oder Partikeln wie Steinchen verhindert wird. Das Abdeckelement kann in diesem Fall mit Stegen an der Wandung oder am Rand des Hohlzylinders befestigt sein.

Mit der Figur 4 wird eine abgewandelte Form des Zugangselements gezeigt. Hierbei weist das Zugangselement 315 am oberen Rand der Wandung 330 des Hohlzylinders eine Abdeckung 300 auf, die durch Öffnungen 320 im Form eines Gitters 310 durchbrochen ist.

In einer weiteren Ausführung der Erfindung ist vorgesehen, dass zusätzlich eine Membran 420 in oder an dem Zugangselement angeordnet ist. Durch diese Membran 420 kann insbesondere verhindert werden, dass Staub oder Dreck durch den Hohlzylinder in den Sensor 100 und an das Sensorelement gelangen kann. Hierbei kann die Membran 420 beispielsweise im unteren Bereich des Zugangselements 110 beziehungsweise 115 angebracht sein.

Wie anhand der Figur 4 gezeigt wird, kann die Membran 420 auch Teil der Befestigung beziehungsweise Abdichtung des Medienkanals 410 im Gehäusedeckel 130 gegenüber dem Außenbereich sein. So kann eine Dichtung 400 insbesondere am Rand der Membran 420 vorgesehen sein, die zusammen mit dem Gehäusedeckel 130 den Innenbereich des Sensors 100 von der Umgebung abdichtet. Dabei kann die Dichtung 400 zusätzlich derart ausgestaltet sein, dass die (mechanische) Schwingungen dämpft, die von außen an dem Zugangselement 110 anliegen.

Die Dichtung 400 kann als Zweikomponenten Dichtung in Form eines Silikons oder Elastomers im Spritzgussverfahren vor dem Zusammenfügen des Zugangselements und dem Gehäusedeckels 130 aufgebracht werden. Dabei kann die Dichtung auch ohne die Verwendung einer Membran direkt auf den Hohlzylinder des Zugangselements aufgebracht werden.

## Patentansprüche

1. Zugangselement (110) für Medien für einen insbesondere mikromechanischen Sensor (100), wobei das Zugangselement (110)
• eingerichtet ist, in oder an einem Medienkanal (410) des Sensors (100) angeordnet zu werden, und
• einen Hohlzylinder (230, 235) aufweist, mit einer oberen ersten Öffnung (250), und
• ein Abdeckelement (200, 205) aufweist, welches im Hohlzylinder (230, 235) angeordnet ist und über wenigstens zwei Stege (210, 215) mit dem Hohlzylinder (230, 235) verbunden ist, und
• eine konische Ausformung (240, 245) an der Innenwand des Hohlzylinders (230, 235) mit einer zweiten Öffnung (270) aufweist.

2. Zugangselement nach Anspruch 1, **dadurch gekennzeichnet, dass** der Hohlzylinder (230, 235) eine untere dritte Öffnung aufweist, wobei die zweite und dritte Öffnung den gleichen Durchmesser aufweisen, wobei insbesondere vorgesehen ist, dass die konische Ausformung (240, 245) bis zur unteren dritten Öffnung ausgebildet ist.

3. Zugangselement nach Anspruch 1 oder 2, **dadurch gekennzeichnet, dass** das Abdeckelement (200, 205) einen Durchmesser aufweist, der größer als die zweite Öffnung (270) ist.

4. Zugangselement nach einem der vorhergehenden Ansprüche, **dadurch gekennzeichnet, dass** das Abdeckelement (200, 205) in der oberen ersten Öffnung (250) angeordnet ist.

5. Zugangselement nach einem der vorhergehenden Ansprüche, **dadurch gekennzeichnet, dass** das Abdeckelement (200, 205) zentral im Hohlzylinder (230, 235) angeordnet ist.

6. Zugangselement nach einem der vorhergehenden Ansprüche, **dadurch gekennzeichnet, dass** der Hohlzylinder (230, 235) ringförmig ausgestaltet ist.

7. Zugangselement nach einem der vorhergehenden Ansprüche, **dadurch gekennzeichnet, dass** an der dritten Öffnung eine Membran (420) angeordnet ist.

8. Sensor (100), insbesondere ein mikromechanischer Sensor, mit einem Zugangselement nach einem der Ansprüche 1 bis 7, wobei der Sensor (100)
• ein Gehäuse (120),
• eine in dem Gehäuse (120) angeordnetes Sensorelement,
• einen Gehäusedeckel (130), und
• ein Zugangselement (110) nach einem der Ansprüche 1 bis 7 aufweist,
wobei der Gehäusedeckel (120)
• einen Medienkanal (410) aufweist, mittels dem eine physikalische und/oder chemische Größe des Mediums in der Umgebung des Sensors (100) an das Sensorelement geführt wird, und
• eine Aufnahme für das Zugangselement (110) aufweist,
wobei das Zugangselement (110)
• in oder an einem Medienkanal (410) des Sensors (100) angeordnet ist, und
• einen Hohlzylinder (230, 235) aufweist, mit einer oberen ersten Öffnung (250), und
• ein Abdeckelement (200, 205) aufweist, welches im Hohlzylinder (230, 235) angeordnet ist und über wenigstens zwei Stege (210, 215) mit dem Hohlzylinder (230, 235) verbunden ist, und
• eine konische Ausformung (240, 245) an der Innenwand des Hohlzylinders (230, 235) mit einer zweiten Öffnung (270) aufweist.

9. Sensor (100) nach Anspruch 8, **dadurch gekennzeichnet, dass** der Hohlzylinder (230, 235) eine untere dritte Öffnung aufweist, die zum Medienkanal (410) hin ausgerichtet ist.

10. Sensor nach Anspruch 8 oder 9, **dadurch gekennzeichnet, dass** zwischen dem Zugangselement (110) und dem Medienkanal (410) eine Membran (420) angeordnet ist, wobei insbesondere vorgesehen ist, dass die Membran (420) an der dritten Öffnung des Zugangselement (110) angeordnet ist.

11. Sensor (100) nach einem der Ansprüche 8 bis 10, **dadurch gekennzeichnet, dass** zwischen Zugangselement (110) und Gehäusedeckel (120) eine Dichtung (400) vorgesehen ist, welche eine dämpfende Wirkung aufweist, wobei insbesondere vorgesehen ist, dass die Dichtung (400) zwischen der Membran (420) und dem Gehäusedeckel (120) vorgesehen ist.
